# EUROPEAN PATENT APPLICATION

(11) **EP 2 266 649 A1**
(43) Date of publication of application: **29.12.2010**
(21) Application number: 10156939.0
(22) Date of filing: 18.03.2010
(51) Int. Cl.: A61M 5/315

(54) **Syringe, particularly for medical and veterinary use, of the multi-chamber, single-use and preloaded type**

(30) Priority: 23.03.2009 IT AR20090017
(71) Applicant: Magrini, Nedo, 52010 Capolona, AR (IT)
(72) Inventor: Magrini, Nedo, 52010, Capolona AR (IT); Regi, Tommaso, 52100, Arezzo (IT)
(74) Representative: Modiano, Micaela Nadia

(57) **Abstract**

A syringe, particularly for medical and veterinary use, of the multichamber, single-use and preloaded type, comprising a containment cylinder (2) forming a compartment (91) for containment and mixing for substances (911) to be injected, which can be mated with a tubular element (3). The containment cylinder (2) is connectable, at a first end (21), to a needle (5) for injections.

The syringe comprises a piston (4) that is inserted in the tubular element (3) and has a containment head (40) that engages jointly, at least in a peripheral portion thereof, an edge (312) of a flared portion (311) formed in an end part (31) of the tubular element (3). The piston (4) also presents at least one flow control element (42) engaging an internal wall (301) of the tubular element (3) and forming, on the tubular element (3), at least one containment chamber (92) for the substances to be mixed.

## Description

The present invention refers to a syringe, particularly for medical and veterinary use, of the multi-chamber, single-use and preloaded type.

In the current state of the art syringes are known, particularly for medical and veterinary use, of the multi-chamber, single-use and preloaded type, having a cylindrical container at its final end connected to a needle and at a second end having an access to the inside, fitted with one or more protrusions for the transit of the substances to be mixed between the chambers that contain them. These chambers, inside the cylinder, are defined by two consecutive flow control elements, while the main containment chamber is comprised between the last flow control element and the final end. Syringes of this type are **characterised in that** they have flow control elements pushed by the action of a plunger acting on the first of these elements, whereas the successive elements proceed in translation moved by the incompressibility of the fluids contained in the chambers inside the cylinder. This translation does not, however, occur jointly.

When each flow control element arrives at one of the protrusions, the substances are free to pass from one containment chamber to the next, until they are all collected in the main chamber and then, by continuing to press on the plunger, they can be injected.

Devices of this type, however, are assembled in a laborious manner and, in particular, they are not easy to use given that the user, by retracting the plunger, cannot draw in air or drops of blood which would verify the correctness of the needle's penetration.

In addition, in zones with a particularly hot climate, under conditions of lengthy storage in non-refrigerated environments, for example in countries in the third world, it can happen that the internal pressure in the cylinder causes a shift of the flow control elements, with the risk of bringing the substances to be mixed into contact prematurely and thus bringing about a chemical reaction that may not be desired.

Finally, these types of syringes do not guard against tampering, be it criminally motivated or accidental.

In the first case, for example, the tampering may be done by malicious parties who want to put substances harmful to the patient's health into the syringe without the operator being able to perceive it, simply by temporarily removing the plunger.

In the second case, the accidental error may occur under extreme conditions in very crowded hospital wards, where injections are prepared all together in the corridor and then injected by medical personnel moving from room to room. Or it may occur in injections performed in the home by family members for a patient who needs treatment with medicines that are similar in appearance.

Another type of multi-chamber, single-use and preloaded syringe implements the transfer of a substance from one chamber to the next by means of piercing of a membrane, with a double-pointed perforating needle which, under the push of the plunger, at one end injects the solution into the patient and at the other end pierces the membrane.

Devices of this type, although effective, are however extremely laborious to assemble and difficult to use. In addition, they can lead to problems of partial mixing of solute and solvent. Finally, a major drawback is that it is not possible to have more than two chambers.

The aim of the present invention is to eliminate the drawbacks noted above in known types of syringes, particularly for medical and veterinary use, of the multi-chamber, single-use and preloaded type, by devising a syringe that guarantees optimum mixing of the solute and solvent substances inside the cylinder while preventing their premature or undesired contact.

Within this aim, an object of the present invention is to devise a syringe, particularly for medical and veterinary use, that ensures maximum hygienic/medical safety, while limiting the risk of infection for the user.

Another object of the invention is to devise a syringe, particularly for medical and veterinary use, that functions in a unidirectional manner, thus not permitting the plunger to be retracted, once the substances to be injected have been mixed, in order to prevent tampering of any kind, be it criminally motivated or accidental.

A further object of the invention is to prevent the substances to be injected from being capable of being adulterated, with or without criminal intent, thus protecting both user and patient.

Another object of the syringe, particularly for medical and veterinary use, according to the invention, is to ensure maximum protection for the substances to be injected and to ensure that their undesired mixing is prevented.

Another object of the invention is to devise a multi-chamber, single-use and preloaded syringe that also permits a suction phase in addition to the injection phase.

A further object of the invention is to devise a syringe, particularly for medical and veterinary use, of the multi-chamber, single-use and preloaded type, with means that are easily obtained on the market and using commonly-used materials, in such a way that the syringe is economically competitive and simple to use and does not require qualified personnel, such as for example nurses, for its use.

This aim, as well as these and other objects which will become better apparent hereinafter, are achieved by a syringe, particularly for medical and veterinary use, of the multi-chamber, single-use and preloaded type, according to the invention, comprising a containment cylinder that forms a compartment for containment and mixing for the substances to be injected and which can be mated with a tubular element, said containment cylinder being connectable, at a first end, to a needle for injections, **characterized in that** it comprises a piston that can be inserted in said tubular element and has a containment head that engages jointly, at least in a peripheral portion thereof, the edge of a flared portion formed in the end part of said tubular element, said piston having at least one flow control element that engages the internal wall of said tubular element and forms, on said tubular element, at least one containment chamber for the substances to be mixed.

Further characteristics and advantages of the invention will become better apparent from the following detailed description of a preferred, but not exclusive, embodiment of the syringe, particularly for medical and veterinary use, of the multi-chamber, single-use and preloaded type, illustrated by way of non-limiting example in the accompanying drawings, wherein:

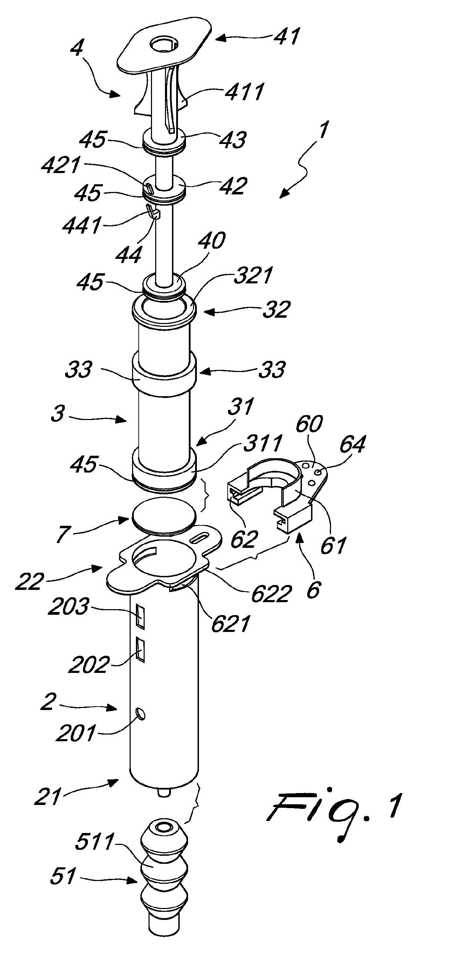

Figure 1 is an exploded perspective view of a syringe, according to the invention;

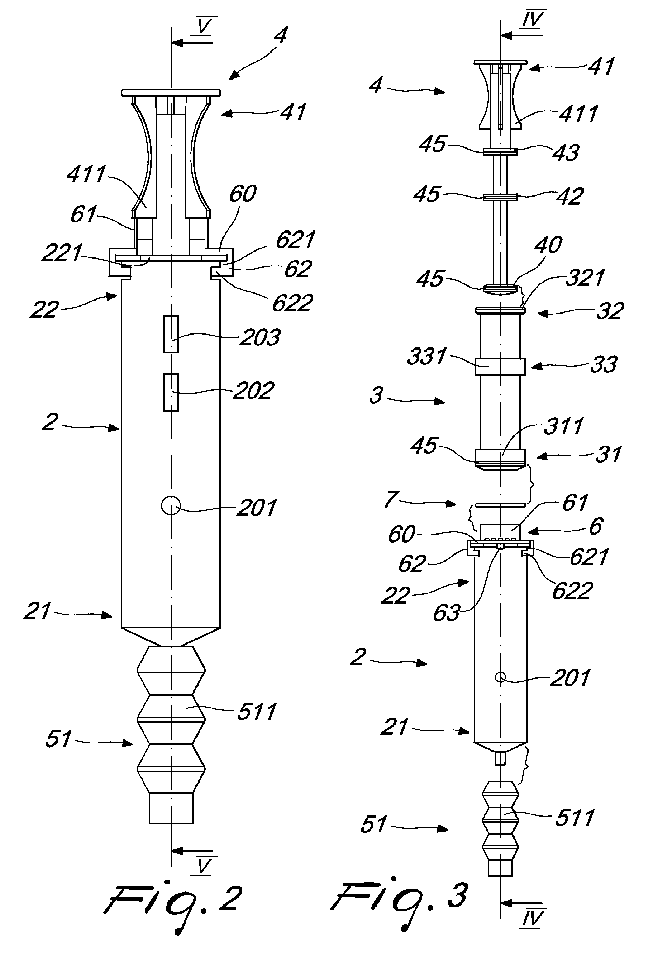

Figure 2 is a side view of the syringe according to the invention;
Figure 3 is an exploded front view of the syringe;
Figure 4 is a perspective view of the device taken along the line IV-IV of Figure 3;

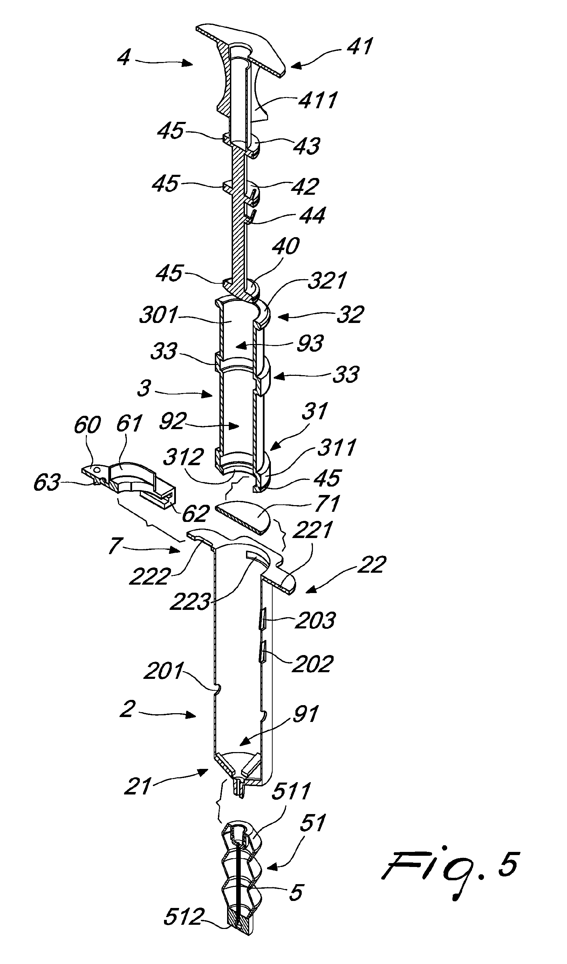

Figure 5 is an exploded view of the device taken along the line V-V of Figure 2;

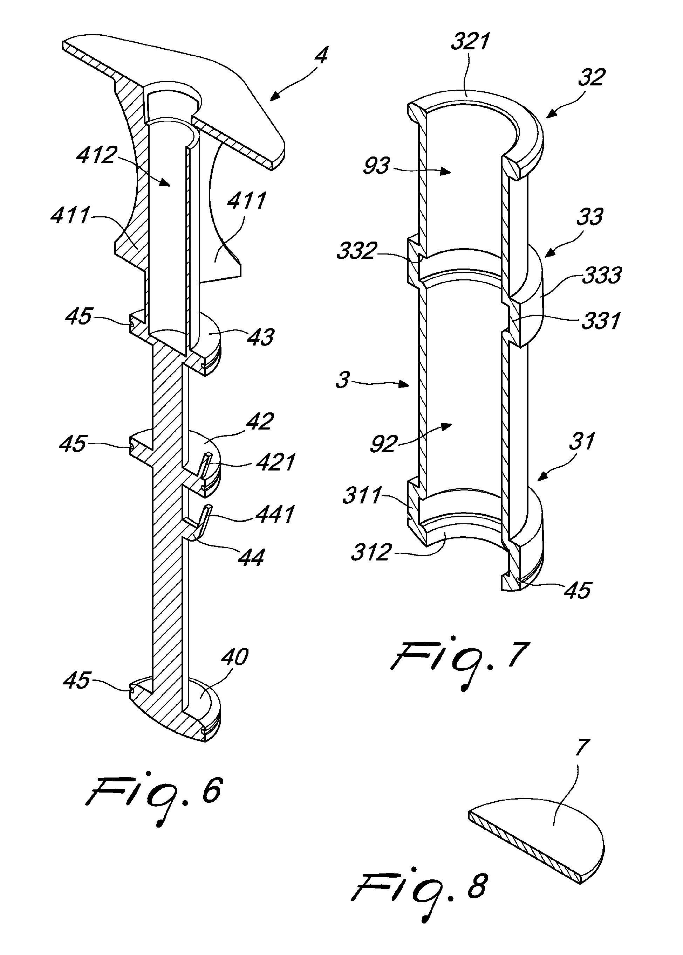

Figure 6 is a perspective sectional view of the piston taken along a plane passing through the axis of the containment cylinder;
Figure 7 is a perspective view of the retention disk sectioned by a plane parallel to the axis of the containment cylinder;
Figure 8 is a perspective sectional view of the tubular element taken along a plane passing through the axis of the containment cylinder;

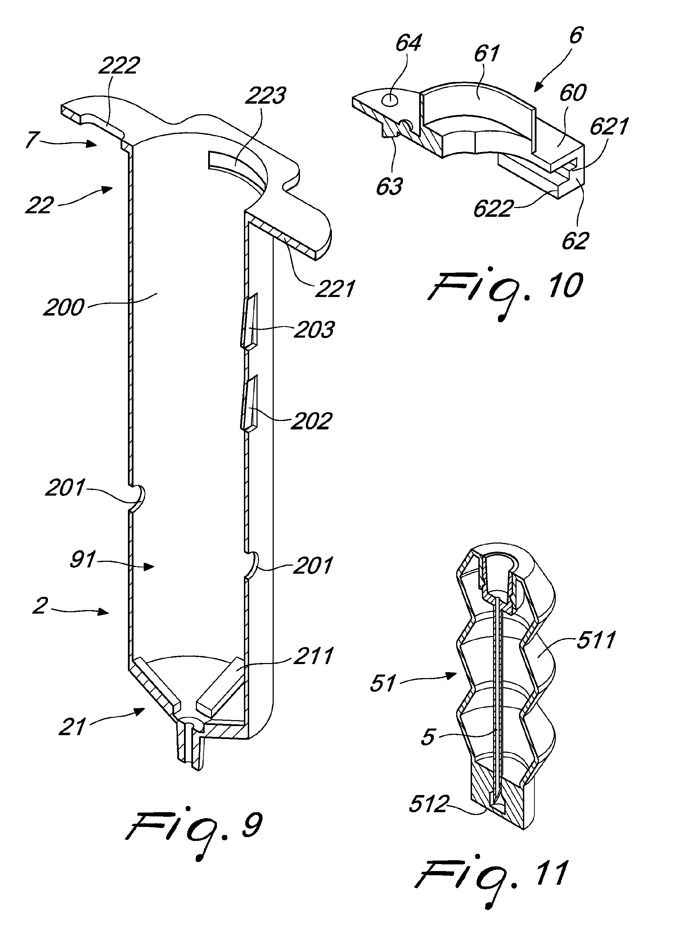

Figure 9 is a perspective sectional view of the containment cylinder taken along a plane passing through the axis of the containment cylinder;
Figure 10 is a perspective sectional view of the safety device taken along a plane passing through the axis of the containment cylinder;
Figure 11 is a perspective sectional view of the needle and of the needle cover taken along a plane passing through the axis of the containment cylinder.

With reference to the figures, a syringe, particularly for medical and veterinary use, of the multi-chamber, single-use and preloaded type, according to the invention, which is generally designated by the reference numeral 1, comprises a containment cylinder 2 that forms a compartment 91 for containment and mixing for the substances to be injected 911, which can be connected, at a first end 21, with a needle for injections 5 and which can be mated with a tubular element 3.

A fundamental characteristic of the invention is that the syringe comprises a piston 4 that can be inserted inside the tubular element 3 and which has a containment head 40 that engages jointly, at least in a peripheral portion thereof, an edge 312 of a flared portion 311 formed in the end part 31 of the tubular element 3. The piston 4 also presents at least one flow control element 42 engaging the internal wall 301 of the tubular element 3 and forming, on such tubular element, at least one containment chamber 92 for the substances to be mixed 921.

Normally the syringe has two grip wings 221 protruding from a second end 22, which lies opposite the first end 21.

In the embodiment shown in the Figures, the syringe comprises three spaces for the containment of the substances: the compartment 91 for the substances to be injected 911 and two containment chambers 92 and 93 for the substances to be mixed, respectively 921 and 931; the piston 4, consequently, has a second flow control element 43 forming the other containment chamber 93.

It can be said therefore that the piston 4 will be fitted with a number of flow control elements equal to the number of containment chambers required, which in turn depend on the substances to be mixed.

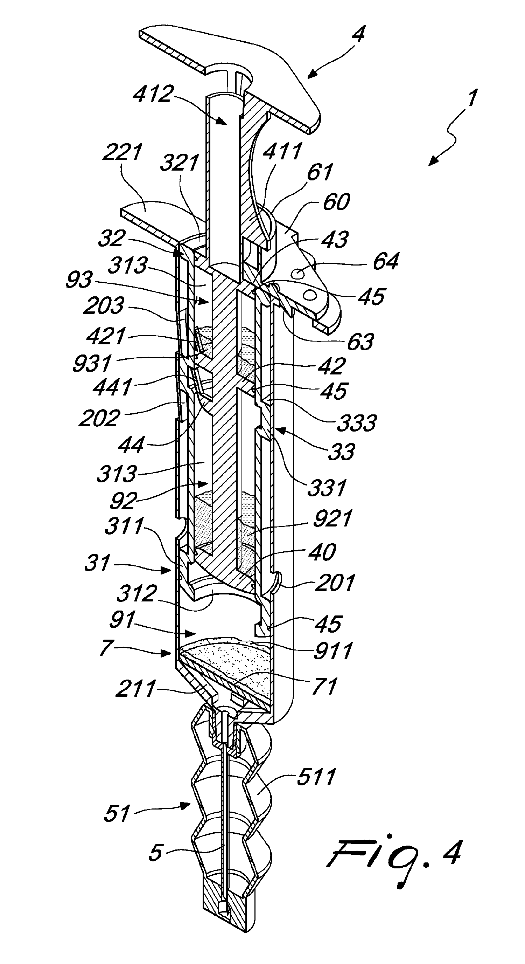

Advantageously, with reference to Figure 4, the tubular element 3 can have, in its central portion 33, at least one radial hollow collar 331 for transferring the substances 931 from the chamber 93 to the chamber 92.

It is possible to implement a number of collars equal to the number of containment chambers in the tubular element 3 in addition to the second chamber 92, to allow simultaneous falling of the substances to be mixed. For example, a syringe with four chambers will be fitted with three radial hollow collars: a compartment for containment and mixing in the containment cylinder and three containment chambers in the tubular element.

The tubular element 3, in addition, can be fitted, at the end 32 opposite the end 31, with a sealing protrusion 321 to prevent any external contact with the substances to be mixed.

Preferably, the side surfaces of the head 40, of the flow control elements 42 and 43, can each have at least one seal ring 45, known per se, which ensures the hermetic sealing of the chambers 92 and 93 when the piston 4 is in contact with the inner wall 313 of the tubular element 3.

Advantageously, there can be locking means for the movement of the piston 4 during the initial suction phase.

It is possible for the apex of the piston 4 to be in contrast with an internal part 332 of the collar 331, before the injection, blocking the movement of the piston itself along the direction of travel of the syringe, in the direction of suction.

Preferably, there is a second locking spacer 421, which protrudes from an end portion of the flow control element 42. This spacer 421 has an apex which in the suction phase contrasts with the internal part 332 of the collar 331, similarly to the configuration of the first spacer 441. In addition, a distance has been chosen between the apex and the first locking spacer 441, which is equal to the stroke allowed to the piston 4 for the suction phase.

Advantageously, for preventing relative movement between the tubular element 3 and the containment cylinder 2 in the initial suction phase, it is possible to have a first stop element 202 associated with the internal wall 200 of the containment cylinder 2. Such stop element is for contrast with the outside surface 333 of the collar 331 and for blocking the relative movement between the tubular element 3 and the containment cylinder 2.

It is also possible to prevent the relative movement between the tubular element 3 and the containment cylinder 2 immediately after the transfer of the substances in the compartment 91, similarly to the method used for the initial locking of the syringe, by associating a second stop element 203 with the internal wall 200 of the containment cylinder 2. The stop element 203 prevents the sliding of the tubular element 3 in the direction of suction.

Advantageously, there is a safety device 6, locking the movement of the piston 4 in the initial phase of injection. Such device can be connected to the containment cylinder 2.

To prevent the syringe from being tampered with before use and to prevent the piston 4 from making any movement along the direction of travel of the syringe when in storage and during manipulation before the injection, the device is interposed between the tubular element 2 and the piston 4.

Advantageously, the safety device 6 comprises a slider 60 which has a contoured tooth 62 which engages the tubular element 2 and a shim 61, associated with a port 41 on the end portion of the piston 4 to facilitate its movement inside the tubular element 3. The shim 61 is arranged opposite an upright 411 that is associated with the piston 4.

The tooth 62 preferably has a first protrusion 621 and a second protrusion 622: the first protrusion 621 engages a surface of the slider 60 and the second protrusion is inserted in a slot 223 that is provided proximately to the end 22 of the cylinder 2.

With the second protrusion 622 it is possible to block the movement of the piston 4 in the direction of injection as well.

To deactivate the safety device 6 and proceed with the injection, there can preferably be a release tab 63 that engages a slot 222 formed on one of the grip wings 221 of the containment cylinder 2. In this way both the disengagement of the protrusion 621 from the surface of the slider 60, and the removal of the second protrusion 622 from the slot 223 are permitted, in such a way as to permit the movement of the slider 60.

On the safety device 6 there may also be a raised portion 64 for increasing the grip of the hand and for assisting the shifting of the slider 60. The raised portion 64, if desired, may bear the contents of the syringe written in Braille.

The cylindrical body 2 can have at least one overpressure hole 201 for the exit of gas from the compartment for containment and mixing 91, in case there is an accumulation of air inside it during the preloading.

In the selected embodiment, the syringe can be fitted with a needle-cover 51 which can be punctured, having a side wall 511 made of a material with oriented fibres, and therefore capable of crumpling very easily following the compression exercised by the piston, when the contact surface 512 of the needle-cover 51 is in contact with the skin of the user.

Advantageously, there is a retention disk 7 resting on raised portions 211 formed on the bottom of the first end 21 of the containment cylinder 2. The retention disk 7 has the particularity of being deformable and therefore in the injection phase it permits the passage of the substances to be injected 911.

In this way a possible obstruction of the channel inside the needle 5 is avoided, for example in case the active ingredient contained in the compartment 91 is constituted by powders of an unusual granulometry.

Producing the containment cylinder 2 from optically transparent material makes it possible to label the tubular element 3, possibly indicating the name of the active ingredient, its identification symbol, the pharmaceutical category and the commercial name. In addition to such label, it is possible to affix marking means indicating the type of medicine (antibiotic, antipyretic, antihistamine etc.), for example by means of a sticker of differing colour or shape.

Finally, for photosensitive active ingredients, the syringe body that contains them can be manufactured in a dark colour.

The syringe thus devised can be packaged in a hermetically-sealed container, ready for use by the user.

Once the safety device 6 has been removed by pulling the tab 63 in the manner described, the piston is pressed in the traditional manner until the edge 312 is engaged by the head 40, jointly connecting the tubular element 3 and the piston 4 to each other. At this point pushing the piston 4 forward also permits the translation of the tubular element 3 with respect to the containment cylinder 2.

When the substances to be injected have all been transferred into the compartment 91, if necessary, the operator can agitate the syringe to favour their mixing, and then proceed with the injection in the classic manner.

When the needle 5 has been inserted in the body of the patient, the user can, if necessary, proceed with the suction of a drop of blood to obtain confirmation of having located a vein, according to medical practice. The device, indeed, permits the backward movement of the piston towards the initial position, but only for a limited stroke equal to the distance between the two elements 441 and 421.

During the injection phase, the substance 911 pushes upon the surface of the retention disk 7 pushed by the action of the piston 4 and the retention disk 7 deforms by bending, thus permitting the substances to be injected to pass inside the needle 5, transiting between the channels formed by the raised portions 211.

Once the injection has been performed, as a further safety procedure, before disposing of the syringe, the needle 5 is removed and inserted in an internal cavity 412 provided for this purpose in the grip 41.

From the above description it can therefore be seen how the invention achieves the intended aim and objects and, in particular, attention is drawn to the fact that a syringe is devised, particularly for medical and veterinary use, of the multi-chamber, single-use and preloaded type, that ensures the optimum mixing of the substances to be injected and guarantees maximum hygienic/medical safety by preventing any premature transfers or contact with external germs and bacteria that can entail the risk of infection.

In particular, having devised a piston fitted with one or more flow control elements forming containment chambers for the substances to be injected that always move jointly with each other prevents an accidental transfer of substances to be injected from one containment chamber to the next, or their undesired mixing, even under conditions of high pressure or storage at high temperatures for long periods of time.

In addition, the devising of a transfer system, the radial hollow collar with which the tubular element is fitted, makes it possible for the substances to be mixed to transit, almost instantaneously and simultaneously, into the compartment in order to be injected.

Another advantage of the invention is that the safety device, combined with the locking means, prevents any movement of the piston, even accidentally, and therefore precludes any attempt at tampering or adulteration, intentional or unintentional, of the syringe.

Another advantage of the syringe, according to the invention, is that, in the tubular element, there is a sealing protrusion that completely closes the containment chambers of the substances to be mixed, limiting the risks of contamination of the syringe contents by dust.

Another advantage of the invention is that the labelling of the outside surface of the tubular element, and/or the marking means fixed thereto, make it possible for the user not to confuse medicines that are similar in appearance, therefore achieving an even higher safety of use.

Another advantage of the syringe, particularly for medical and veterinary use, of the multi-chamber, single-use and preloaded type, is that of also having enabled, while guaranteeing all of the aforementioned safety measures, a suction phase, limited by the stroke set in the design phase to be equal to the distance between the first and the second locking spacer, in such a way as to allow the operator to verify whether a vein or air has been located.

Another advantage of the syringe, according to the invention, consists of having devised a system of depressurisation of the compartment for containment and mixing which makes it possible to have a loading phase that is free from risks of overloading.

Another advantage of the syringe thus provided is ensuring minimum external contact as a result of the needle-cover able to be crumpled, which allows the operator to mix the substances in sequence, preparing a plurality of injections, and then proceeding to use the syringes without worrying about needle/air contact, especially in locations that are not sterile.

In addition, the ability to extract the needle after use to place it inside the grip of the piston, safe from further manipulations, limits the possibility of accidental puncture wounds which involve a serious risk of infection.

Another advantage of the syringe, according to the invention, is due to the presence of the retention disk resting on the bottom of the end of the containment cylinder 2 which does not have an effect on the correct functioning of the syringe and at the same time prevents the obstruction, full or partial, of the hole in the needle.

Another advantage of the syringe, particularly for medical and veterinary use, of the multi-chamber, single-use and preloaded type, consists in being entirely similar to traditional syringes, the use of which does not require specialist personnel or a longer learning period than the one necessary to learn how to perform injections with traditional syringes, made of common and easily obtained materials and means.

In practice it has been found that the syringe thus described permits a mixing of the substances contained inside it that is perfectly controlled by the operator, thus eliminating all risk of tampering, adulteration or contamination, thus guaranteeing maximum hygiene and cleanliness.

The invention, thus conceived, is susceptible of numerous modifications and variations, all of which are within the scope of the appended claims.

In addition, all the details may be replaced by other technically equivalent elements.

In practice the materials employed, as well as the dimensions and the contingent shapes, may be any according to requirements, as long as they are consistent with the appended claims.

The disclosures in Italian Patent Application No. AR2009A000017 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A syringe, particularly for medical and veterinary use, of the multi-chamber, single-use and preloaded type, comprising a containment cylinder (2) that forms a compartment (91) for containment and mixing for substances to be injected (911) and that can be mated with a tubular element (3), said containment cylinder (2) being connectable, at a first end (21), to a needle for injections (5), **characterized in that** it comprises a piston (4) that can be inserted in said tubular element (3) and has a containment head (40) that engages jointly, at least in a peripheral portion thereof, an edge (312) of a flared portion (311) formed in an end part (31) of said tubular element (3), said piston (4) having at least one flow control element (42) that engages an internal wall (301) of said tubular element (3) and forms, on said tubular element (3), at least one containment chamber (92) for the substances to be mixed (921).

2. The syringe according to claim 1, **characterized in that** it comprises at least one radial hollow collar (331), which is formed in the central portion (33) of said tubular element (3), for the transfer of said substances to be mixed from each one of said at least one containment chamber to said containment and mixing compartment.

3. The syringe according to claim 1, **characterized in that** it comprises locking means for the movement of said piston (4) for suction.

4. The syringe according to claim 3, **characterized in that** said locking means comprise a first locking spacer (441), which is fixed to a tab (44) that protrudes from said piston (4) and engages an internal part (332) of said radial hollow collar (331).

5. The syringe according to claim 1, **characterized in that** it comprises a safety device (6) for locking the movement of said piston (4) in the direction of injection in the initial step, which is connected to said containment cylinder (2).

6. The syringe according to claim 5, **characterized in that** said safety device (6) comprises a slider (60), which has a contoured tooth (62) for engaging said tubular element (3) and a shim (61), which is associated with a port (41) that is connected to an end portion of said piston (4), said shim (61) being arranged opposite an upright (411) that is associated with said piston (4).

7. The syringe according to claim 6, **characterized in that** said contoured tooth (62) comprises a first protrusion (621) and a second protrusion (622), said first protrusion (621) engaging a surface of said slider (60) and said second protrusion (622) being designed for insertion in a slot (223) that is provided proximate to a second end (22) of said containment cylinder (2), which lies opposite said first end (21).

8. The syringe according to claim 7, **characterized in that** said safety device comprises a release tab (63) that engages a slot (222) formed on a grip wing (221) of said containment cylinder (2) for the disengagement of said first protrusion (621) from said surface of said slider (60) and the removal of said second protrusion (622) from said slot (223).

9. The syringe according to claim 2, **characterized in that** it comprises a first stop element (202), which is associated with an internal wall (200) of said containment cylinder (2), for contrast with an outer surface (333) of said radial hollow collar (331), for locking the relative movement between said tubular element (3) and said containment cylinder (2).

10. The syringe according to claim 4, **characterized in that** it comprises a second locking spacer (421), which protrudes from an end portion of said flow control element (42), which has an apex for contrast with said internal part (332) during suction and has a distance from said first locking spacer (441) that is equal to the stroke allowed to said piston (4) for said suction step.

11. The syringe according to claim 1, **characterized in that** it comprises a retention disk (7), which rests on raised portions (211) formed on the bottom of said one first end (21) of said containment cylinder (2) and is deformable, during injection, for the passage of said substances to be injected (911).
